# EUROPEAN PATENT APPLICATION

(11) **EP 2 141 498 A1**
(43) Date of publication of application: **06.01.2010**
(21) Application number: 08450097.4
(22) Date of filing: 02.07.2008
(51) Int. Cl.: G01N 33/569

(54) **Cellular COPD diagnosis**

(71) Applicant: Apoptec AG, 1010 Vienna (AT)
(72) Inventor: Ankersmit, Jan Hendrik, 1040 Vienna (AT)
(74) Representative: Sonn & Partner Patentanwälte

(57) **Abstract**

Method for diagnosing the risk of a human subject to develop chronic obstructive pulmonary disease (COPD) comprising the steps of:
- providing a sample from a human subject,
- determining the amount of CD4+CD28nu11 T-cells in said sample,
- diagnosing the risk to develop COPD when the amount of CD4+CD28nu11 T-cells is reduced compared to the amount of CD4+CD28nu11 T-cells in healthy human subjects.

## Description

The present invention relates to a method for diagnosing the risk of a human subject to develop chronic obstructive pulmonary disease (COPD).

Chronic Obstructive Pulmonary Disease (COPD) is one of the leading causes of death worldwide. In 2020, only ischemic heart disease and cerebrovascular disease will account for a higher mortality among the world's population. Prevalence and hospitalization rates have inclined dramatically over the past years. Several studies have shown a strong correlation between tobacco smoking and the development of COPD although not every smoker develops the clinical features of COPD (Higenbottam T et al. (1980) Lancet 315:409-411). The pathogenesis is characterized by airflow obstruction due to airway remodelling and aberrant inflammation. COPD comprises chronic bronchitis and emphysema, both conditions characterized by tissue destruction. Airflow limitation is slowly progressive, leading to dyspnoea and limitations of physical exercise capacities. However, impairment is not restricted to the lungs, as COPD patients are also at higher risk for systemic failures including cardiovascular diseases. Diagnosis of airway obstruction according to the guidelines of the Global Initiative for Chronic Obstructive Lung Diseases (GOLD) requires the use of spirometry. A postbronchodilator FEV1/FVC (forced expiratory volume in one second/forced vital capacity) ratio of less than 70% indicates an irreversible airflow obstruction, and is therefore considered to be the main parameter for the diagnosis of COPD (Global Strategy for Diagnosis, Management, and Prevention of COPD. Global Initiative FOR Chronic Obstructive Lung Disease, 2007, www.goldcopd.com). Currently, patients are classified into GOLD stages according to spirometry data and clinical presentation. The detection of serum markers indicating disease activity is of special interest in the diagnostic and therapeutic process.

Although smoking is widely accepted as the major risk factor for the development of the disease, descriptions of specific pathogenetic mechanisms remain vague. For decades, neutrophils and macrophages - as part of the innate immunity - were considered pivotal in the airway remodelling process occurring in patients with COPD. Recent reports have challenged this pathognomonic concept by evidencing increased CD8+ and CD4+ T-cells - as part of the adaptive immune system - in bronchoalve-olar lavage (BAL) and sputum analyses of COPD patients. These T-lymphocytes contained higher levels of perforin and revealed cytotoxic activity as compared to cells of healthy donors or non-COPD smokers. Antigenic stimulation causes a rapid expansion of antigen-specific T cells that expand to large clonal size. This expansion is counterbalanced by a pre-programmed clonal contraction. This process is robust and usually suffices to maintain a diverse memory T-cell compartment. However, chronic antigen exposure, e.g. HIV and CMV virus infection, elicits expansion of monoclonal T-cell populations. Furthermore, age contributes profoundly on T-cell homeostasis.

The only method used in the clinical practice for diagnosing COPD is spirometry, which is a pulmonary function test measuring lung function, specifically the measuring of the amount (volume) and/or speed (flow) of air that can be inhaled and exhaled. However, such tests are also used to diagnose other pulmonary diseases like asthma and pulmonary fibrosis. Therefore, such a method cannot function as the sole test to reliable diagnose COPD. Therefore, the physicians consider also symptoms like dyspnea, chronic cough or sputum production, and/or a history of exposure to risk factors for diagnosing COPD. It is evident that the use of such methods in diagnosing COPD or in discriminating various forms of COPD may result in a false diagnosis, so that the patient cannot utilise the best form of therapy right from the beginning of the disease.

Therefore, it is an object of the present invention to provide methods and means which allow for unequivocally diagnosing COPD in a human subject from the beginning of the disease or even for determining the risk of a human subject to develop COPD.

The present invention relates to a method for diagnosing the risk of a human subject to develop chronic obstructive pulmonary disease (COPD) comprising the steps of:
- providing a sample from a human subject,
- determining the amount of CD4+CD28null T-cells in said sample,
- diagnosing the risk to develop COPD when the amount of CD4+CD28null T-cells is reduced compared to the amount of CD4+CD28null T-cells in healthy human subject.

Another aspect of the present invention relates to a method for diagnosing chronic obstructive pulmonary disease (COPD) in a human subject comprising the steps of:
- providing a sample from a human subject, determining the amount of CD4+CD28null T-cells in said sample,
- diagnosing COPD when the amount of CD4+CD28null T-cells is increased compared to the amount of CD4+CD28null T-cells in healthy human subjects.

It turned out that the determination of the amount of CD4+CD28null cells in a human subject can function as marker which allows to diagnose COPD, in particular COPD stages I/II and III/IV in a human subject. The marker allows even to determine the risk of a human subject to develop COPD in the future. Not all humans which are considered as being at risk to develop COPD due to their lifestyle (e.g. humans subjected to smoke, smokers etc.) will come down with COPD. Therefore, the diagnosis that a human subject is at risk to develop COPD is very useful in the prevention of COPD. Furthermore, it is also useful to diagnose COPD and to discriminate between COPD stages I/II and III/IV. The discrimination between both COPD stages is useful for applying a distinct therapy. Consequently the determination of CD4+CD28null T-cells is also useful to determine the progress of a therapy or the progress of the disease as such.

In the method according to the present invention CD4+CD28null cells are determined which do not comprise at their surface CD28. CD4+ T-cells comprising a reduced amount of CD28 on their surface are excluded from this method. Furthermore, CD4+CD28null T-cells are a distinct type of cell which are not able to produce CD28. This definition does not include other CD4+ T-cells which produce only a reduced amount of CD28 compared to regularly occurring CD4+CD28+ T-cells. In Gadgil A. et al. (Proc Am Thorac Soc 3 (2006): 487-488) such CD4+ T-cells are described. In these cells the expression of CD28 is down-regulated, although these cells still express CD28.

The marker used in the method of the present invention may be used singularly or in combination with any other markers used to diagnose COPD in humans being at risk to develop COPD known in the art.

CD4 is a glycoprotein expressed on the surface of T helper cells and other cells like regulatory T cells, monocytes, macrophages, and dendritic cells. CD28 is one of the molecules expressed on T cells that provide co-stimulatory signals, which are required for T cell activation. CD28 is the receptor for B7.1 (CD80) and B7.2 (CD86). When activated by Toll-like receptor ligands, the B7.1 expression is upregulated in antigen presenting cells (APCs). The B7.2 expression on antigen presenting cells is constitutive. **(**P. Sansoni et al. Exp. Gerontology 43 (2008): 61-65**.** FA. Arosa. Immunol and Cell Biol. 80 (2002): 1-13**)**

Replicatively stressed CD4+ T-cells undergo multiple phenotypic and functional changes. The most widely acknowledged phenotypic change is the loss of the co-stimulatory surface marker CD28. Expansion of CD4+ T cells and loss of CD28 are presumably senescent. This has been described in several autoimmune diseases such as diabetes mellitus, rheumatoid arthritis, Wegener's granulomatosis, multiple sclerosis and ankylosing spondylitis. CD4+CD28null cells are clonally expanded and are known to include autoreactive T-cells, implicating a direct role in autoimmune disease. Theses expanded CD4+ clonotypes are phenotypically distinct from the classic T-helper-cells. Due to a transcriptional block of the CD28 gene, clonally expanded CD4+ T-cells lack surface expression of the major co-stimulatory molecule CD28. CD4+CD28null T-cells release large amounts of interferon-γ (IFN-γ) and contain intracellular perforin and granzyme B, providing them with the ability to lyse target cells. Their outgrowth into large clonal populations may be partially attributed to a defect in down-regulating Bcl-2 when deprived of T-cell growth factors. In the absence of the CD28 molecule, these unusual CD4+ T-cells use alternative co-stimulatory pathways. Several of these functional features in CD4+CD28- T-cells are reminiscent of natural killer (NK) cells.

Like NK cells, CD4+CD28- T-cells are cytotoxic and can express NK-cell receptors, e.g. CD94 and CD158. NK cells are closely regulated by a family of polymorphic receptors that interact with major histocompatibility complex (MHC) class I molecules, resulting in signals that control NK-mediated cytotoxicity and cytokine production. MHC class I-mediated triggering of the full-length NK cell receptors transduces a dominant inhibitory signal that blocks the cytolytic activity and cytokine release of NK cells. These receptors also contain highly homologous members that have truncated cytoplasmatic domains and transmit activating signals.

In order to classify the severity of COPD spirometric parameters are used. These parameters allow to classify the severity of COPD into four stages (see Table A). Spirometry is essential for diagnosis and provides a useful description of the severity of pathological changes in COPD. Specific spirometric cut-points (e.g., post-bronchodilator FEV₁/FVC ratio < 0.70 or FEV₁ < 80, 50, or 30% predicted) are used to determine the COPD stages I to IV.

**Table A: Spirometric Classification of COPD (according to www.goldcopd.com). Severity Based on Post-Bronchodilator FEV₁.**

| | |
|---|---|
| **Stage I:** Mild | FEV₁/FVC < 0.70 |
| | FEV₁ 80% predicted |
| **Stage II:** Moderate | FEV₁/FVC < 0.70 |
| | 50% FEV₁ < 80% predicted |
| **Stage III:** Severe | FEV₁/FVC < 0.70 |
| | 30% FEV₁ < 50% predicted |
| **Stage IV:** Very Severe | FEV₁/FVC < 0.70 |
| | FEV₁ < 30% predicted or FEV₁ < 50% predicted plus chronic respiratory failure |

| | |
|---|---|
| FEV₁: forced expiratory volume in one second; FVC: forced vital capacity; respiratory failure: arterial partial pressure of oxygen (PaO₂) less than 8.0 kPa (60 mm Hg) with or without arterial partial pressure of CO₂ (PaCO₂) greater than 6.7 kPa (50 mm Hg) while breathing air at sea level. | |

Methods for determining FEV₁ and FVC, which can be used to systematise COPD (see Table A), are well-known in the art (see e.g. Eaton T, et al. Chest (1999) 116:416-23; Schermer TR, et al. Thorax (2003) 58:861-6; Bolton CE, et al. Respir Med (2005) 99:493-500).

The impact of COPD on an individual patient depends not just on the degree of airflow limitation, but also on the severity of symptoms (especially breathlessness and decreased exercise capacity). There is only an imperfect relationship between the degree of airflow limitation and the presence of symptoms. The characteristic symptoms of COPD are chronic and progressive dyspnea, cough, and sputum production. Chronic cough and sputum production may precede the development of airflow limitation by many years. This pattern offers a unique opportunity to identify smokers and others at risk for COPD, and intervene when the disease is not yet a major health problem.

Conversely, significant airflow limitation may develop without chronic cough and sputum production. Although COPD is defined on the basis of airflow limitation, in practice the decision to seek medical help (and so permit the diagnosis to be made) is normally determined by the impact of a particular symptom on a patient's lifestyle.

**Stage I:** Mild COPD - Characterized by mild airflow limitation (FEV₁/FVC < 0.70; FEV₁ 80 % predicted). Symptoms of chronic cough and sputum production may be present, but not always. At this stage, the individual is usually unaware that his or her lung function is abnormal.

**Stage II:** Moderate COPD - Characterized by worsening airflow limitation (FEV₁/FVC < 0.70; 50% FEV₁ < 80% predicted), with shortness of breath typically developing on exertion and cough and sputum production sometimes also present. This is the stage at which patients typically seek medical attention because of chronic respiratory symptoms or an exacerbation of their disease.

**Stage III:** Severe COPD - Characterized by further worsening of airflow limitation (FEV₁/FVC < 0.70; 30% FEV₁ < 50% predicted), greater shortness of breath, reduced exercise capacity, fatigue, and repeated exacerbations that almost always have an impact on patients' quality of life.

**Stage IV**: Very Severe COPD - Characterized by severe airflow limitation (FEV₁/FVC < 0.70; FEV₁ < 30% predicted or FEV₁ < 50% predicted plus the presence of chronic respiratory failure). Respiratory failure is defined as an arterial partial pressure of O₂ (PaO₂) less than 8.0 kPa (60 mm Hg), with or without arterial partial pressure of CO₂ (PaCO₂) greater than 6.7 kPa (50 mm Hg) while breathing air at sea level. Respiratory failure may also lead to effects on the heart such as cor pulmonale (right heart failure). Clinical signs of cor pulmonale include elevation of the jugular venous pressure and pitting ankle edema. Patients may have Stage IV: Very Severe COPD even if the FEV₁ is > 30% predicted, whenever these complications are present. At this stage, quality of life is very appreciably impaired and exacerbations may be life threatening.

The term "at risk to develop COPD", as used herein, refers to a pool of human individuals which are subjected to environmental threats or which may have a genetic predisposition to develop COPD. Factors which support the formation of COPD include genetic predisposition, exposure to particles like tobacco smoke, occupational dusts (organic and inorganic), indoor air pollution from heating and cooking with biomass in poorly vented dwellings and Outdoor air pollution, lung growth and development, oxidative stress, gender, age, respiratory infections, so-cioeconomic status, nutrition and comorbidities. Humans, which are at risk to develop COPD may suffer from chronic cough, chronic sputum production and normal spirometry. However humans suffering from these symptoms do necessarily progress on to COPD stage I.

As used herein, the term "healthy human subjects" or "healthy human" refers to humans who do not suffer from COPD or any other pulmonary disease. Furthermore, these individuals did not have any severe pulmonary disease in their life. "Healthy humans" do also not include humans who are regularly exposed to risk factors, like smoke or other noxious substances.

According to the present invention the amount of the marker in "healthy humans" is determined by quantifying this marker in at least 5, 10, 15, or 20 "healthy humans".

The sample of the human subject is preferably blood, more preferably heparinized blood.

In order to determine the amount of CD4+CD28null cells antibodies directed to CD4 and CD28 are preferably used. The use of antibodies allows to specifically detect and optionally quantify cells which present on their surface antigens like CD4 and CD28.

The term "antibody", as used herein, refers to monoclonal and polyclonal antibodies or fragments thereof capable to bind to an antigen. Other antibodies and antibody fragments, such as recombinant antibodies, chimeric antibodies, humanized antibodies, antibody fragments such as Fab or Fv fragments, as well as fragments selected by screening phage display libraries, and the like are also useful in the methods described herein.

Methods for preparation of monoclonal as well as polyclonal antibodies are well established (Harlow E. et ah, 1988. Antibodies. New York: Cold Spring Harbour Laboratory). Polyclonal antibodies are raised in various species including but not limited to mouse, rat, rabbit, goat, sheep, donkey, camel and horse, using standard immunization and bleeding procedures. Animal bleeds with high titres are fractionated by routine selective salt-out procedures, such as precipitation with ammonium sulfate and specific immunoglobulin fractions being separated by successive affinity chromatography on Protein-A-Sepharose and leptin-Sepharose columns, according to standard methods. The purified polyclonal as well as monoclonal antibodies are then characterised for specificity. Such characterization is performed by standard methods using proteins labeled with a tracer such as a radioisotope or biotin in competition with increasing levels of unlabeled potential cross-reactants for antibody binding. Binding studies are further evaluated by other standard methods such as the well-established sodium dodecyl sulphate-polyacrylamide gel electrophoresis (SDS-PAGE) and Western immunoblot methods under reducing and non-reducing conditions.

Monoclonal antibodies are prepared according to well established standard laboratory procedures ("Practice and Theory of Enzyme Immunoassays" by P. Tijssen (hi Laboratory Techniques in Biochemistry and Molecular Biology, Eds: R.H. Burdon and P.H. van Kinppenberg; Elsevier Publishers Biomedical Division, 1985)), which are based on the original technique of Kohler and Milstein (Kohler G., Milstein C. Nature 256:495, 1975). This technique is performed by removing spleen cells from immunized animals and immortalizing the antibody producing cells by fusion with myeloma cells or by Epstein-Barr virus transformation, and then screening for clones expressing the desired antibody, although other techniques known in the art are also used. Antibodies are also produced by other approaches known to those skilled in the art, including but not limited to immunization with specific DNA.

Antibodies binding specifically to CD4 and CD28 are preferably employed in flow cytometry, in particular fluorescence-activated cell sorting (FACS), in order to determine the amount of CD4+CD28null cells in a sample. The antibodies are used to label cells which comprise CD4 and/or CD28 on their surface.

In order to detect labelled cells in the flow cytometer the antibodies binding to CD4 and CD28 are preferably tagged with FITC, Alexa Fluor 488, GFP, CFSE, CFDA-SE, DyLight 488, PE, Per-CP, PE-Alexa Fluor 700, PE-Cy5 (TRI-COLOR), PE-Cy5.5, PI, PE-Al-exa Fluor 750, PE-Cy7, APC and APC-Cy7.

In order to diagnose COPD or the risk to develop COPD it is advantageous to define cut-off levels above or beneath which the disease can be diagnosed. According to a preferred embodiment of the present invention the amount of CD4+CD28null cells in a healthy human subject is less than 2.5%, preferably less than 2.3%, and more than 1.7%, preferably more than 1.8% of the total CD4+ cell population. The amount of CD4+CD28null cells of the total CD4+ cell population varies in "healty humans" in between 1.7 and 2.5%.

The total CD4+ T-cell population is determined by methods known in the art. According to another preferred embodiment of the present invention the risk to develop COPD is diagnosed when the amount of CD4+CD28null cells in the sample is at least 10%, preferably at least 20%, reduced compared to the amount of CD4+CD28null cells in healthy humans. Of course the amount of CD4+CD28null cells in the sample may also be decreased by at least 15%, 25%, 30%, 40%, 50%, 60%, 70%, 80%, 90% or 100%. According to a particularly preferred embodiment, the amount of CD4+CD28null T-cells in humans at risk to develop COPD is between 0.8 and 1.6% (preferably between 0.9 and 1.5%, 1 and 1.4%) of the total CD4+ cell population.

According to a further preferred embodiment, the amount of CD4+CD28null T-cells in a human subject suffering from COPD is more than 2.7%, preferably more than 2.8%, even more preferably more than 3%, of CD4+CD28null T-cell of the total CD4+ cell population.

The method according to the present invention may be applicable for other mammals, such as horse, dog, cat and cattle.

Another aspect of the present invention relates to a method for discriminating between COPD stage I/II and COPD stage III/IV in a human subject comprising the steps of:
- providing a sample from a human subject suffering from COPD,
- determining the amount of CD4+CD28null cells in said sample,
- diagnosing COPD stage I/II when the amount of CD4+CD28null cells is reduced compared to the amount of CD4+CD28null cells determined in a sample from a human subject suffering from COPD stage III/IV, or
- diagnosing COPD stage III/IV when the amount of CD4+CD28null cells is increased compared to the amount of CD4+CD28null cells determined in a sample from a human subject suffering from COPD stage I/II.

The COPD markers disclosed herein are also suited to discriminate between human subjects suffering from COPD stage I/II and COPD stage III/IV as defined above. The discrimination between these COPD stages is important to determine the therapy. For patients with few or intermittent symptoms (stage I and II), for instance, use of a short-acting inhaled bronchodilator as needed to control dyspnea is sufficient. If inhaled bronchodilators are not available, regular treatment with slow-release theophylline should be considered. In humans whose dyspnea during daily activities is not relieved despite treatment with as-needed shortacting bronchodilators, adding regular treatment with a long-acting inhaled bronchodilator is recommended. In humans suffering COPD stages III/IV regular treatment with inhaled glucocorticosteroids reduces the frequency of exacerbations and improves health status. In these humans, regular treatment with an inhaled glucocorticosteroid should be added to long-acting inhaled bronchodilators. For humans suffering from COPD stage III/IV surgical treatments and/or long term oxygen should be considered if chronic respiratory failure occurs.

The reference values which allow to discriminate between COPD stages I/II and III/IV can be assessed by determining the respective amounts of CD4+CD28null cells in a pool of samples obtained from humans suffering COPD stages I/II and III/IV. Such a pool may comprise samples obtained from at least 5, preferably at least 10, more preferably at least 20, humans suffering from COPD and for which the various COPD stages have been diagnosed by alternative methods (e.g. spirometry). As mentioned above the amount of CD4+CD28null T-cells of the total CD4+ cell population varies in human subjects between 1.7 and 2.5%. Humans being at risk to develop COPD comprise 0.8 to 1.6% CD4+CD28null T-cells of the total CD4+ T-cell population. Humans suffering from COPD stage I/II comprise 2.7 to 4.5% (preferably 2.8 to 4.3%) CD4+CD28null T-cells of the total CD4+ T-cell population. Humans suffering from COPD stage III/IV comprise 5 to 12% (preferably 6 to 10%) CD4+CD28null T-cells of the total CD4+ T-cell population.

In an alternative embodiment of the present invention COPD stages I/II can be discriminated from COPD stages III/IV by determining the amount of IFN-γ.

A further aspect of the present invention relates to a method for monitoring the progress of chronic obstructive pulmonary disease (COPD) in a human subject comprising the steps of:
- providing a sample from a human subject,
- determining the amount of CD4+CD28null cells in said sample,
- comparing the amount of CD4+CD28null cells in the sample of said human subject with the amount of CD4+CD28null cells in a sample from said human subject determined in an earlier sample of said human subject.

The markers disclosed herein can also be used to monitor the progress of COPD and the progress of a COPD therapy. Such a method involves the comparison of the amount of CD4+CD28null cells in samples obtained from a human at different time intervals. The results obtained from said method allow the physician to set an appropriate therapy.

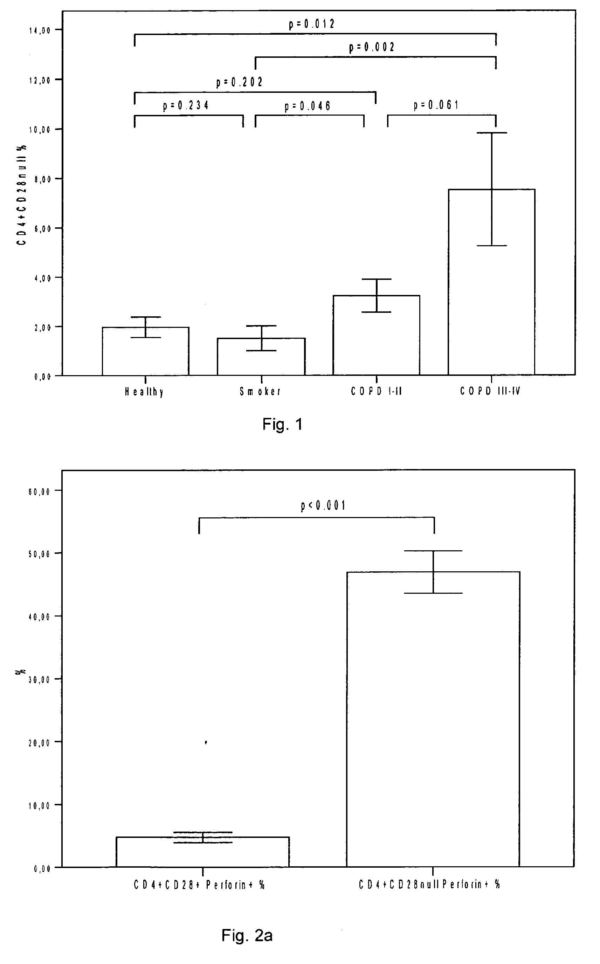

The present invention is further illustrated by the following figures and example, however, without being restricted thereto. Fig. 1 shows percentage of CD4+CD28null cells in the peripheral blood flow. Results are expressed as mean +/- SEM.

Fig. 2 shows a subset of CD4+ T cells lacking co-stimulatory CD28 contained intracellular cytolytic proteins perforin (a) and granzyme B (b). Results are expressed as mean +/- SEM.

Fig. 3 shows CD4/CD28null cells showing significantly increased surface expression of NK cell receptors CD94 and CD158. Results are expressed as mean +/- SEM.

Fig. 4 shows scatterplots showing correlations of CD4+CD28null % of CD4+ and FEV1%, MEF50%, and MEF25%, Spearman's correlation coefficients and p-values are given.

Fig. 5 shows ROC curve for the predicition of COPD in the subgroup of smokers based on the CD4+CD28null % measurement.

### EXAMPLES:

### Example 1:

### Material and Methods

### Patients:

A total number of 64 volunteers, at least 40 years old, participated in this trial. Healthy non-smokers (n=15); healthy smokers (n=14) and smokers meeting the GOLD diagnostic criteria for COPD I&II (n=19) and COPD III&IV (n=16) 23 were recruited. COPD patients with acute exacerbation as defined by the guidelines of the WHO and the Global Initiative for Chronic Obstructive Lung Disease (GOLD) (Global Strategy for Diagnosis, Management, and Prevention of COPD. Global Initiative FOR Chronic Obstructive Lung Disease, 2007, www.goldcopd.com) within 14 days before study entry were excluded. Additional exclusion criteria were a history of asthma, autoimmune diseases or other relevant lung diseases (e.g., lung cancer, known al-antitrypsin deficiency). Furthermore, all patients were free from known coronary artery disease, peripheral artery disease, and carotid artery disease. Height and weight (Seca; Vogel and Halke, Germany) were measured and the body mass index (BMI) was determined. Pulmonary function (FEV1, FVC, and FEV1/ FVC ratio) was measured using the same model spirometer (AutoboxV6200, SensorMedics, Austria). Measurements were made before and - if criteria for airflow obstruction were met - 15 - 30 minutes after inhaling of 200 µg salbutamol. Arterial blood gases (PaO₂, PaCO₂) were obtained at rest while breathing room air in a sitting position. Measurement of arterial blood gases was performed with an ABL 510 gas analyzer (Radiometer, Denmark). Results are expressed as absolute values and as percentages of predicted values for age, sex and height, according to the European Community for Steel and Coal prediction equations **(**Quanje PH et al. Eur Respir J Suppl 16 (1993): 5-40**).** Predicted normal values were derived from the reference values of the Austrian Society of Pulmonary Medicine (Harnoncourt K et al. Österreich. Ärztetg. 37 (1982): 1640-1642).

### Flow Cytometry Analysis

Heparinized blood samples were incubated on ice with fluorochrome-labelled antibodies. Prior to antibody incubation, erythrocytes were lysed by addition of BD FACS Lysing Solution (Becton Dickinson, Becton Drive, Franklin Lakes, New Jersey, USA). Cells were then stained with FITC-conjugated anti-CD4 (BD Biosciences Pharmingen, USA), PE-labelled anti-CD158 (R&D Systems, USA), PE-Cy5-labelled anti-CD28 (Biolegend, USA) and PE-conjugated anti-CD94 (eBioscience, USA) at various combinations. Stained cells were analyzed using a Cytomics FC 500 flow cytometer (Beckman Coulter, USA). For intracellular staining, PE-conjuagted antibodies directed against perforin and granzyme B (BD Biosciences Pharmingen, USA; Serotec, Germany) were used and incubated with pre-stained cells after permeabilization of the cell membrane with saponin solution.

### Enzyme-linked immunosorbent assays (ELISA)

ELISA technique (BenderMedSystems, Austria) was used to quantify levels of IL-1β, TNF-α, IFN-γ, and IL-10 in serum samples obtained after centrifugation of whole blood.

96-well plates were coated with a monoclonal antibody directed against the specific antigen and incubated over night at 4°C. After a washing step, plates were blocked with assay buffer for two hours. Following another washing step, samples and standards with defined concentrations of antigen were incubated as described by the manufacturer. Plates were then washed and incubated with enzyme-linked polyclonal antibodies. TMB substrate solution was applied after the appropriate time of incubation and another washing step. Color development was then monitored using a Wallac Multilabel counter 1420 (PerkinElmer, USA). The optical density (O.D.) values obtained were compared to the standard curve calculated from O.D. values of standards with known concentrations of antigen.

### Stimulation of freshly prepared peripheral blood mononuclear cells

Freshly prepared peripheral blood mononuclear cells (PBMCs) were separated by standard Ficoll densitiy gradient centrifugation. Cells were then washed twice in PBS, counted and transferred to a 96-well flat-bottom plate at 1*10⁵ cells per well in 200µL serum free Ultra Culture Medium (Cambrex Corp., USA) containing 0.2% gentamycinsulfate (Sigma, USA) and 0.5% β-Mercaptoethanol (Sigma, USA) 1% L-Glutamin (Sigma, USA). Anti-CD3 (CD3) (10µg/mL) or phytohemagglutinin (PHA) (7µg/mL) were added and plates were transferred to a humidified atmosphere (5% CO₂ 37°C) for 18 hours. Supernatants were harvested and stored at -20°C.

### Quantification of IFN-γ, TNF-α, and IL-12 in supernatants

ELISA technique (BenderMedSystems, Austria) was used to quantify levels of IFN-γ, TNF-α, and IL-12 in supernatants of stimulated cells as described above.

### Statistical methods

Pairwise comparisons of the primary endpoint CD4+CD28null % of CD4+ between healthy non-smokers, healthy smokers, COPD I&II and COPD III&IV patients were performed with non-parametric Wilcoxon tests. To adjust for multiple testing (6 group comparisons), additionally the non-parametric Nemenyi-Damico-Wolfe-Dunn (NDWD) tests controlling the family wise error rate across the 6 comparisons was performed (function one-way-test in the coin R-package). Parametric 95% confidence intervals for the mean CD4+CD28null percentages in each group were computed. For the between group comparison of IFN-γ, TNF-α, and IL-12 ex vivo CD3 and PHA measurements only the multiplicity adjusted analysis with the NDWD-test is reported. Correlations of serum cytokine levels with parameters of lung function were calculated using the spearman's correlation coefficient.

The correlations of the percentage of CD4+CD28null cells with FEV1 % of vital capacity, MEF50 % of predicted value and MEF25 % of predicted value was assessed with spearman's correlation coefficient. Prevalence of perforin, granzyme B and expression of CD94 and CD158 was compared between CD4+CD28null and CD4+CD28+ cells using Wilcoxon Signed Rank tests. Additionally, parametric 95% confidence intervals for the mean percentages for each variable are given.

In the subgroup of smokers a logistic regression with dependent variable COPD (yes/ no) and independent variable CD4CD28null % was performed. To account for an outlying observation, the square root of the percentages was used in this analysis. To assess the predictive capacity of the percentage of CD4CD28null an ROC curve with its AUC was computed.

### Results

### Demographic characteristics of study patients

Demographic characteristics of patients are depicted in Table 1 and 2. Healthy non-smokers, healthy smokers, GOLD classified COPD I&II, COPD III&IV were included. In all groups a similar number of patients was included and age and sex were equally distributed.

**Table 1: Clinical characteristics (severity of airflow obstruction was determined using lung function test [LFT] in all subjects; COPD patients meeting the GOLD diagnostic criteria for COPD). Data are given as mean if not otherwise stated.**

| **Subject Category** | **Healthy** | **Healthy Smoker** | **COPD GOLD I-IV** | **COPD GOLD I&II** | **COPD GOLD III&IV** |
|---|---|---|---|---|---|
| **n** | 15 | 14 | 35 | 19 | 16 |
| **Male/Female** | 10/5 | 7/7 | 20/15 | 10/9 | 10/6 |
| **Age** | 57.20 | 56.64 | 59.60 | 60.68 | 58.31 |
| **(SD)** | 12.50 | 9.17 | 8.01 | 7.39 | 8.75 |
| | | | | | |
| **Lung Function Test** | | - | | | |
| **FVC (L)** | 4.55 | 3.84 | 2.80 | 3.33 | 2.14 |
| (SD) | 0.94 | 0.66 | 1.08 | 1.06 | 0.70 |
| **FEV1 (%)** | 105.37 | 94.40 | 52.76 | 70.21 | 30.67 |
| (SD) | 17.11 | 11.96 | 23.71 | 13.33 | 12.66 |
| **FEV1/VC (%)** | 76.80 | 75.95 | 51.18 | 61.74 | 37.80 |
| (SD) | 7.85 | 3.99 | 16.83 | 8.36 | 15.33 |
| **MEF 50 (%)** | 100.67 | 87.64 | 27.29 | 39.42 | 11.93 |
| (SD) | 28.92 | 21.45 | 18.68 | 15.93 | 6.60 |
| **MEF 25 (%)** | 103.53 | 75.71 | 29.71 | 37.37 | 20.00 |
| (SD) | 33.89 | 31.33 | 15.31 | 16.19 | 5.94 |

| | | | | | |
|---|---|---|---|---|---|
| (Abbreviations: COPD - Chronic Obstructive Pulmonary Disease; FVC - Forced Vital Capacity; FEV1 - Forced Expiratory Volume in 1 second; MEF= Maximal Expiratory Flow; SD - Standard Deviation) | | | | | |

**Table 2: Clinical characteristics and smoking status of all study subjects. Data are given as mean if not otherwise stated. (SD - standard deviation)**

| **Subject Category** | **Healthy** | **Healthy Smoker** | **COPD GOLD I-IV** | **COPD GOLD I-II** | **COPD GOLD III-IV** |
|---|---|---|---|---|---|
| **Smoking History** | | | | | |
| **Never-smoker (n)** | 15 | 0 | 0 | 0 | 0 |
| **Ex-smoker (n)** | 0 | 3 | 7 | 4 | 3 |
| **Current-smokers (n)** | 0 | 11 | 28 | 15 | 13 |
| **Pack Years** | 0 | 34 | 45.8 | 47.3 | 44.0 |
| (SD) | 0 | 25.2 | 30.6 | 29.7 | 32.6 |
| **Body Weight (kg)** | 71.6 | 76.4 | 80.4 | 79.7 | 81.1 |
| (SD) | 13.9 | 8.6 | 21.6 | 16.7 | 27.2 |
| **Body Height (cm)** | 172.7 | 168.7 | 169.2 | 167.7 | 171.2 |
| (SD) | 10.9 | 8.1 | 10.5 | 12.1 | 7.9 |

### CD4+CD28null cells show increased occurrence in patients suffering from COPD

To test whether CD4+CD28null cells are increased in patients with chronic obstructive pulmonary disease, blood samples using multi-stain flow cytometry were evaluated. Fig. 1 and Table 4 illustrate percentages of CD4+CD28null cells of the total CD4+ cell population. The COPD III&IV group showed significantly increased values compared to the healthy non-smoker and healthy smoker group (Wilcoxon test: p=0.012, p=0.002). Additionally, we observed a significant difference between the COPD I&II group and the healthy smoker group (Wilcoxon test: p=0.046). After correcting for multiplicity only the differences between the COPD III&IV and the healthy groups remained significant.

### Unstimulated CD4+CD28null cells contain cytolytic proteins perforin and granzyme B

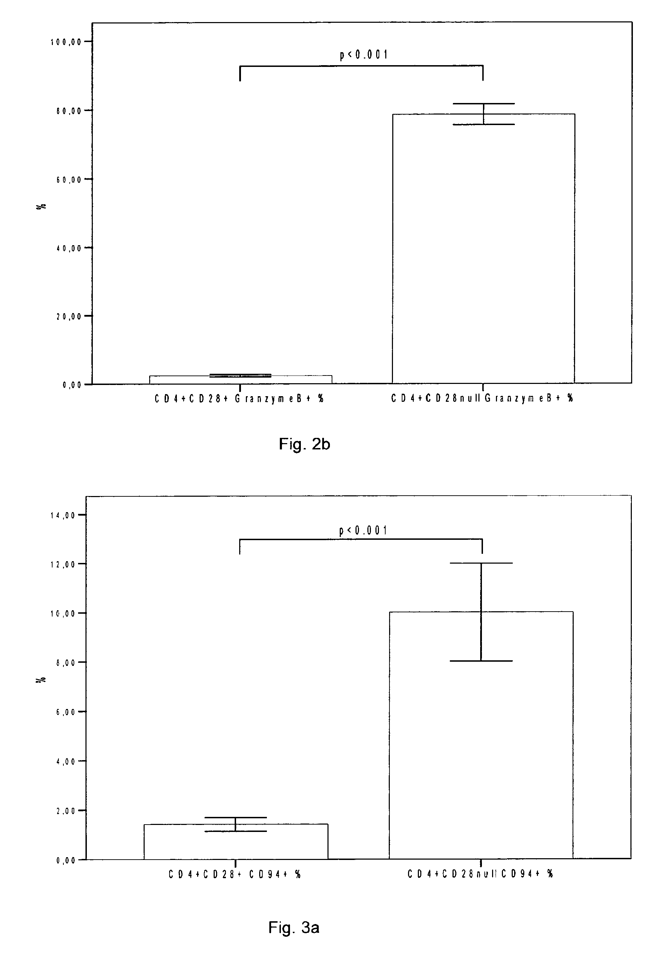

To evaluate the intra-cytoplasmic content of cytolytic proteins perforin and granzyme B in CD4+ cells, flow cytometric analysis of blood samples was performed after co-incubation with saponin solution and intracellular staining. Content of perforin was more prevalent in CD4+CD28null cells as compared to CD4+CD28+ cells. Fig. 2 (a). (46.13% [39.34-52.91] versus 4.68% [3.04-6.32], p<0.001; all, mean [95% CI]) Positive staining for intracellular granzyme B in CD4+CD28null cells was more frequent than in CD4+CD28+ cells. Figure 2 (b). (78.63% [72.65-84.61] versus 2.36% [1.63-3.11], p<0.001; all, mean [95% CI]).

### Increased prevalence of natural killer cell receptors on CD4+CD28null cells

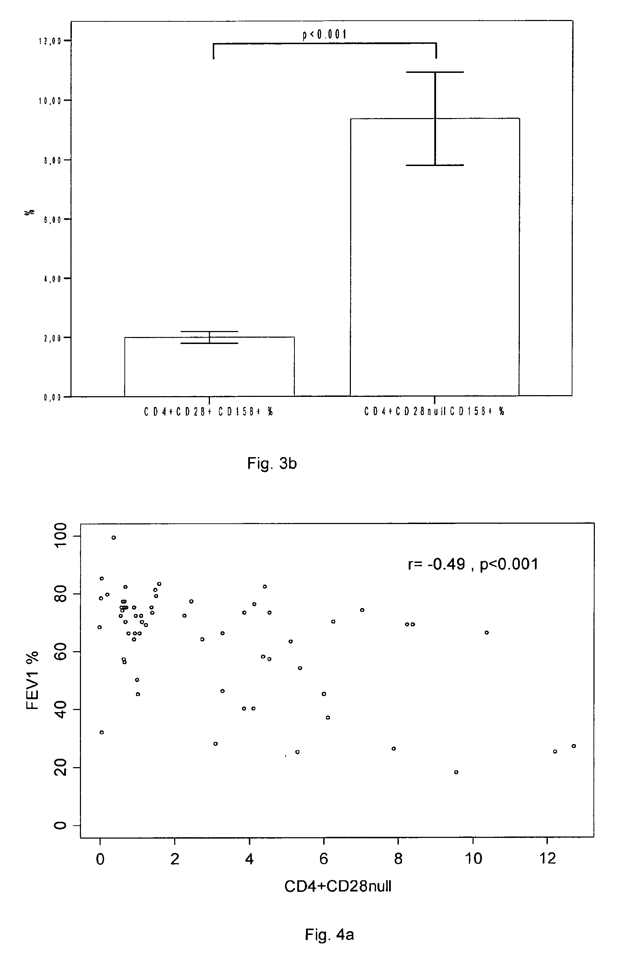

Flow cytometry analysis was used to evaluate expression of CD94 and CD158 on the surface of CD4+ cells. Fig. 3 (a-b) shows increased expression of surface antigens CD94 and CD158 on CD4+CD28null cells (CD94, 10.00% [6.04-13.97] versus 1.41% [0.85-1.97], p<0.001; CD158, 9.35% [6.22-12.47] versus 2.00% [1.61-2.39], p<0.001; all, mean [95% CI]).

### Percentage of CD4+CD28null cells correlates negatively with routine parameters of spirometry

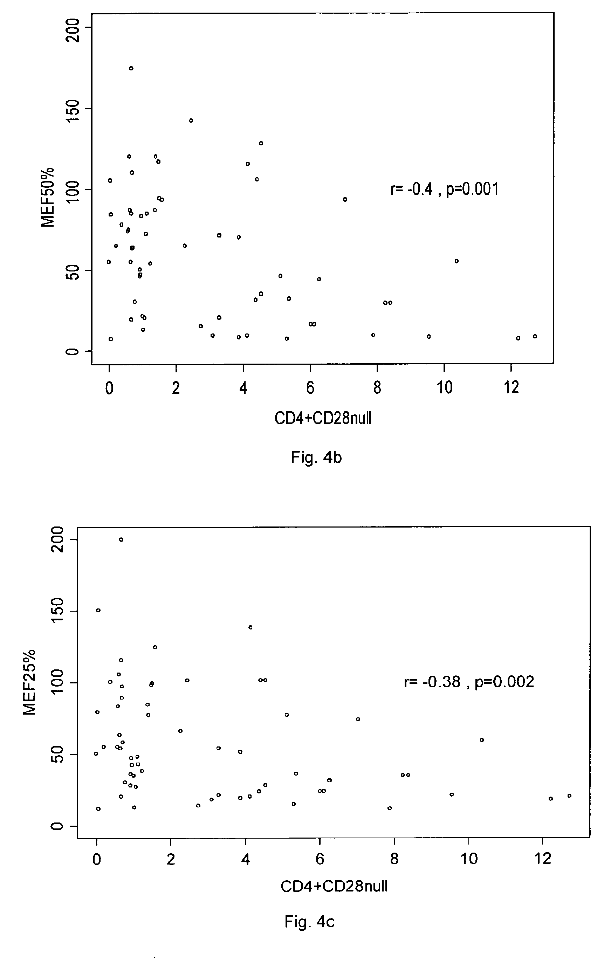

For verification of our flow cytometry data with routine clinical data, the percentage of CD4+CD28null was correlated with FEV1 % of vital capacity, MEF50 % of predicted value and MEF25 % of predicted value. All parameters showed a statistically significant negative correlation with percentage of CD4+CD28null cells. (Spearman's correlation coefficients: FEV1%, R=-0.49, p<0.001; MEF50%, R=-0.40, p=0.001; MEF25%, -0.38, p=0.002). Fig. 4 (a-c).

### Prediction Capacity of the percent of CD4+CD28null cells for COPD in smokers

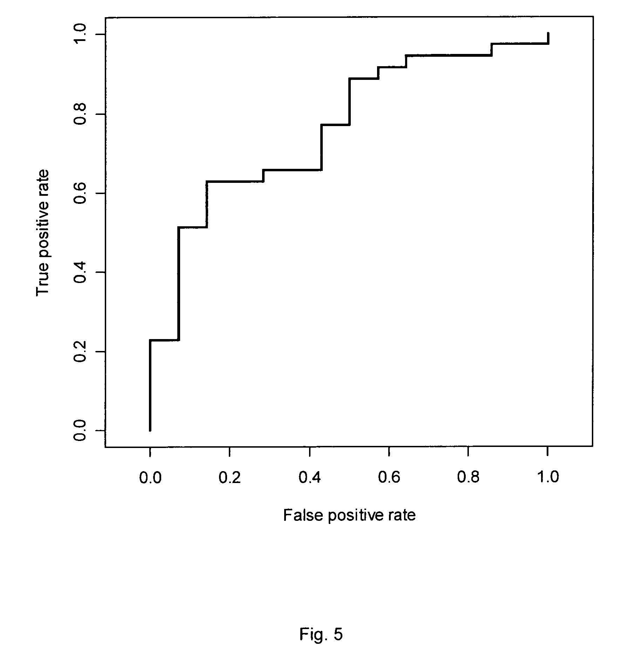

In the logistic regression analysis for the subset of smokers the independent variable percent of CD4+CD28null cells showed a significant association with COPD (p=0.012). The corresponding ROC curve (Fig. 5) has an area under the curve of AUC=0.76.

### Correlations of serum cytokine concentrations (IL-1β, TNF-α, IFN-γ, and IL-10) with FEV1%, MEF50%, MEF25%

Table 3 embraces the results of non-parametric correlations of serum cytokines IL-1β, TNF-α, IFN-γ, and IL-10 with routine lung function parameters.

**Table 3: Correlations of serum cytokine levels with parameters of lung function test. Coefficients and p-values were calculated for all patients enrolled in the present example.**

| **Correlation** | **Coefficient** | **p-value** |
|---|---|---|
| | | |
| IFN-γ - FEV1% | 0.538 | <0.001 |
| IFN-γ - MEF50% | 0.556 | <0.001 |
| IFN-γ - MEF25% | 0.489 | <0.001 |
| TNF-α - FEV1% | 0.334 | 0.008 |
| TNF-α - MEF50% | 0.337 | 0.007 |
| TNF-α - MEF25% | 0.309 | 0.014 |
| IL-1β - FEV1% | 0.291 | 0.022 |
| IL-1β - MEF50% | 0.284 | 0.026 |
| IL-1β - MEF25% | 0.267 | 0.036 |
| IL-10 - FEV1% | 0.325 | 0.01 |
| IL-10 - MEF50% | 0.328 | 0.009 |
| IL-10 - MEF25% | 0.300 | 0.018 |

### Stimulated PBMCs of patients suffering from early stage COPD produce increased levels of IFN-γ and TNF-α ex vivo

To verify the functional activity of peripheral blood mononuclear cells blastogenesis assays were performed using lymphocyte-specific anti-CD3 and phytohemagglutinin. This analysis was performed for 7 patients per group (except of the COPD III&IV group where only 5 patients were included). Groupwise means and 95% confidence intervals are given in Table 4.

**Table 4: The table shows the percentage of CD4+CD28null cells in the peripheral blood flow. Furthermore, cytokine expression in supernatants of PBMCs stimulated with either anti-CD3 or PHA is described. All data are given as mean.**

| **Subject Category** | **Healthy** | **Healthy Smoker** | **COPD GOLD I&II** | **COPD GOLD III&IV** |
|---|---|---|---|---|
| **CD4+CD28null % of CD4+** | 1.96 | 1.5 | 3.22 | 7.53 |
| **IFN-**γ **CD3 pg/mL** | 272 | 240 | 440 | 328 |
| **IFN-**γ **PHA pg/mL** | 116 | 91 | 375 | 134 |
| **TNF-**α **CD3 pg/mL** | 922 | 731 | 1234 | 1508 |
| **TNF-**α **PHA pg/mL** | 1096 | 777 | 2465 | 1144 |
| **IL-12 CD3 pg/mL** | 93 | 63 | 72 | 42 |
| **IL-12 PHA pg/mL** | 44 | 33 | 78 | 17 |

Supernatants of patients with COPD I&II showed marginally significant increased levels of IFN-γ as compared to healthy smokers (NDWD test: CD3: p=0.049; PHA: p=0.062); Concentrations of the healthy group and of patients with COPD III&IV were lower but showed no significant difference to the COPD I&II group. None of the remaining pairwise comparisons was statistically significant.

The COPD I&II group showed significantly elevated levels of TNF-α (PHA) levels compared to healthy smokers (NDWD test: p=0.001) and non-smokers (NDWD test: p=0.047) and marginally significant elevated levels compared to COPD III&IV patients (NDWD test: p=0.054). None of the remaining pairwise comparisons was statistically significant. For TNF-α (CD3) no significant differences between groups were observed. For IL-12 (PHA) we observed higher levels in the COPD I&II group compared to the other groups. However, only the difference to the COPD III&IV group reached statistical significance. Concentrations of IL-12 (CD3) showed no significance between group differences.

### Conclusion

The total number of lymphocytes circulating in the blood and their subset distribution is under strict homeostatic control. In this example it was shown that patients with COPD evidence a profound change in the representation of functionally and phenotypically distinct subsets of CD4+ T cells. Clonogenic CD4+ T-cells with characterized loss of co-stimulatory CD28, and intracellular storage of the cytolytic proteins granzyme B and perforin might be causal for continuing systemic inflammatory state in COPD patients even after cessation of smoking. The basic mechanisms causing replacement of other CD4+ T-cells by CD4+CD28null clonotypes are incompletely understood. However, phenotypic and functional analyses of CD4+CD28null T-cells show that they are related to NK cells and represent a population of NK-like T-cells 26. It was found that CD4+CD28null T-cells express MHC class I-recognizing receptors of the Ig superfamily (CD94, CD158). The present data corroborate the concept that CD4+CD28null T-cells share multiple features with NK cells and may combine functional properties of innate and adaptive immunity.

To prove relevant immune functions peripheral blood mononuclear cells (PBMCs) of study groups were separated and activated via specific and unspecific T-cell stimulation in vitro. It could be evidenced that systemic white blood cells derived from COPD GOLD I&II secreted augmented levels of IFN-γ and TNF-α - cytokines that are known to increase macrophage and dendritic cell activity - as compared to controls. This observation is particularly interesting as this in vitro phenomenon was observed only in patients at the initial stage of disease progression (GOLD stages I&II), indicating a specific role of NK-like T-cells in triggering initial lung tissue destruction. This in vitro finding led to explore whether systemic serum levels of IL-1β, TNF-α, IFN-γ, and IL-10 were elevated in COPD patients without recent exacerbation. These proteins, however, did not increase with impairment of spirometric parameters. In consequence, it was investigated if presence of systemic clonogenic CD4+CD28null T-cells is relevant for diagnosing COPD. A logistic regression analysis was performed which allowed to show that presence of systemic CD4+CD28null T-cells was highly predictive for diagnosis of COPD.

Whatever competing mechanism is causative for COPD, the presence of systemic chronic inflammation in COPD has been associated with a variety of co-morbidities including, cachexia, osteoporosis, and cardiovascular diseases. The relationship between COPD and cardiovascular diseases is especially germane as over half of patients with COPD die of cardiovascular causes. It was also demonstrated in elegant studies that patients with unstable angina (UA) are characterized by a perturbation of functional T cell repertoire (CD4+CD28null) with a bias toward increased IFN-γ production as compared to patients with stable angina (SA). The fact that CD4+CD28null cells have been found in the blood of patients with UA and in extracts from coronary arteries containing unstable plaques seems to support the idea that the expansion of circulating CD28-lacking CD4+ cells in UA not only sustains systemic inflammation but also plays a pathogenic role in atherosclerosis and tissue degeneration, most probably via the synthesis of high levels of pro-inflammatory cytokines. Interestingly, in direct relevance to this it was also discovered that patients suffering from rheumatoid arthritis (RA) have increased levels of circulating CD4+CD28null T-cells that are directly related to preclinical atherosclerotic changes, including arterial endothelial dysfunction and carotid artery wall thickening. The observation of T cell pool perturbation might be relevant in explaining the previously observed long-term cardiovascular risk in COPD.

Chronic antigen exposure, e.g. through contents of tobacco smoke, leads to loss of CD28 and up-regulation of NK cell receptors expression on T-cells in potentially genetically susceptible patients. This induced immunological senescence is accompanied by a dysregulation of apoptosis inducing signals, e.g. Bcl-2, fostering longevity of cytotoxic T-cells and increased secretion of IFN-γ and TNF-α upon in vitro T-cell triggering. However, it remains ambiguous why in vitro increment of IFN-γ and TNF-α production was evidenced only in COPD I&II after T-cell-specific triggering. From these data it seems clear why cessation of smoking in COPD patients may not fully attenuate the progressive cell based inflammatory process in lung tissue.

## Claims

1. Method for diagnosing the risk of a human subject to develop chronic obstructive pulmonary disease (COPD) comprising the steps of:
- providing a sample from a human subject,
- determining the amount of CD4+CD28null T-cells in said sample,
- diagnosing the risk to develop COPD when the amount of CD4+CD28null T-cells is reduced compared to the amount of CD4+CD28null T-cells in healthy human subjects.

2. Method for diagnosing chronic obstructive pulmonary disease (COPD) in a human subject comprising the steps of:
- providing a sample from a human subject determining the amount of CD4+CD28null T-cells in said sample,
- diagnosing COPD when the amount of CD4+CD28null T-cells is increased compared to the amount of CD4+CD28null T-cells in healthy human subject.

3. Method according to claim 1 or 2, **characterised in that** the sample is blood, preferably heparinized blood.

4. Method according to any one of claims 1 to 3, **characterised in that** the amount of CD4+CD28null cells is determined by using antibodies directed to CD4 and CD28.

5. Method according to any one of claims 1 to 4, **characterised in that** the amount CD4+CD28null T-cells is determined by flow cytometry, in particular fluorescence-activated cell sorting (FACS).

6. Method according to any one of claims 1 to 5, **characterised in that** the amount of CD4+CD28null cells in a healthy human subject is between 1.7 and 2.5% of the total CD4+ T-cell population.

7. Method according to any one of claims 1 to 6, **characterised in that** the risk to develop COPD is diagnosed when the amount of CD4+CD28null cells in the sample is at least 10%, preferably at least 20%, reduced compared to the amount of CD4+CD28null cells in healthy human subjects.

8. Method according to any one of claims 1 and 3 to 7, **characterised in that** the risk to develop COPD is diagnosed when the amount of CD4+CD28null T-cells in the sample is between 0.8 and 1.6% of the total CD4+ T-cell population.

9. Method according to any one of claims 2 to 7, **characterised in that** COPD is diagnosed when the amount of CD4+CD28null T-cells in the sample is more than 2.7% of the total CD4+ T-cell population.

10. Method for discriminating between COPD stage I/II and COPD stage III/IV in a human subject comprising the steps of:
- providing a sample from a human subject suffering from COPD,
- determining the amount of CD4+CD28null cells in said sample,
- diagnosing COPD stage I/II when the amount of CD4+CD28null cells is reduced compared to the amount of CD4+CD28null cells determined in a sample from a human subject suffering from COPD stage III/IV, or
- diagnosing COPD stage III/IV when the amount of CD4+CD28null cells is increased compared to the amount of CD4+CD28null cells determined in a sample from a human subject suffering from COPD stage I/II.

11. Method according to claim 10, **characterised in that** COPD stage I/II is diagnosed when the amount of CD4+CD28null T-cells is 2.7 to 4.5% of the total CD4+ T-cells population.

12. Method according to claim 10 or 11, **characterised in that** COPD stage III/IV is diagnosed when the amount of CD4+CD28null T-cells is 5 to 12% of the total CD4+ T-cell population.

13. Method for monitoring the progress of chronic obstructive pulmonary disease (COPD) in a human subject comprising the steps of:
- providing a sample from a human subject,
- determining the amount of CD4+CD28null cells in said sample,
- comparing the amount of CD4+CD28null cells in the sample of said human subject with the amount of CD4+CD28null cells in a sample from said human subject determined in an earlier sample of said human subject.
